(19) Europäisches Patentamt
European Patent Office
Office européen des brevets

(11) **EP 1 915 977 B1**

(12) **EUROPÄISCHE PATENTSCHRIFT**

(45) Veröffentlichungstag und Bekanntmachung des Hinweises auf die Patenterteilung:
**15.04.2015 Patentblatt 2015/16**

(51) Int Cl.:
*A61F 13/56* (2006.01)    *A61F 13/62* (2006.01)

(21) Anmeldenummer: **06022450.8**

(22) Anmeldetag: **27.10.2006**

(54) **Absorbierender Inkontinenzartikel mit verbessertem Verschlusssystem**

Absorbent incontinence article with an improved fastening system

Article absorbant pour incontinence ayant un système de fermeture amélioré

(84) Benannte Vertragsstaaten:
**AT BE BG CH CY CZ DE DK EE ES FI FR GB GR HU IE IS IT LI LT LU LV MC NL PL PT RO SE SI SK TR**

(43) Veröffentlichungstag der Anmeldung:
**30.04.2008 Patentblatt 2008/18**

(73) Patentinhaber: **Paul Hartmann AG**
**89522 Heidenheim (DE)**

(72) Erfinder:
• **Beckert, Susanne, Dr.**
**89073 Ulm (DE)**
• **Kesselmeier, Rüdiger, Dr.**
**89542 Herbrechtingen (DE)**

(56) Entgegenhaltungen:
EP-A1- 0 800 808        EP-A1- 2 008 539
EP-A2- 1 048 236        WO-A1-2007/001815
DE-A1-102004 021 353    DE-A1-102004 053 469
US-A- 5 549 592         US-A1- 2003 119 404

**Beschreibung**

Die vorliegende Erfindung betrifft eine absorbierende Inkontinenzwindel mit einem Rückbereich, einem Vorderbereich und einem dazwischen liegenden Schrittbereich und mit erstem und zweitem Seitenrand, und mit einem Hauptteil mit einer in Gebrauch dem Körper zugewandten Innenseite und einer in Gebrauch dem Körper abgewandten Außenseite, wobei der Hauptteil einen Saugkörper und ein Backsheet auf der dem Körper abgewandten Seite des Saugkörpers umfasst, und wobei der Saugkörper eine geringere Breite aufweist als das Backsheet, und mit an den ersten und zweiten Seitenrand angefügten diskreten Seitenteilen. Die Inkontinenzwindel ist für Erwachsene vorgesehen und ist als Wegwerfwindel ausgebildet, das heißt zum einmaligen Gebrauch bestimmt.

Derartige Inkontinenzwindeln sind beispielsweise auch aus der WO2004/105668A1 bekannt.

Bei derartigen Inkontinenzwindeln können die erwähnten Seitenteile aus einem anderen Material gebildet sein als der Hauptteil. Beispielsweise können die Seitenteile, die häufig auch als "Ohren" der Inkontinenzwindel bezeichnet werden, atmungsaktiv, insbesondere luft- und wasserdampfdurchlässig ausgebildet werden, wohingegen der Hauptteil, der häufig auch als Chassis bezeichnet wird, flüssigkeitsundurchlässig, insbesondere feuchtigkeitsundurchlässig ausgeführt sein kann. Zum Schließen der Inkontinenzwindel werden die vorzugsweise unlösbar am Rückbereich angefügten Seitenteile auf die Bauchseite des Benutzers geschlagen und dort entweder mit der Außenseite des Vorderbereichs des Hauptteils oder mit der Außenseite der Seitenteile des Vorderbereichs lösbar verbunden.

Insoweit eine derartige Inkontinenzwindel mit mechanischen Verschlusshilfen ausgestattet wird, resultiert das Problem, dass für die üblicherweise an den hinteren Seitenteilen angeordneten meist als Kletthaken ausgeführten Verschlusshilfen ein entsprechender Landebereich an der Außenseite des Vorderbereiches der Windel vorgesehen sein muss, der mit den Kletthaken in Eingriff bringbar sein muss.

Üblicherweise wird die Außenseite des Hauptteils derartiger Inkontinenzwindeln allerdings durch ein Folienmaterial gebildet, um ein Austreten von Flüssigkeit durch den Saugkörper hindurch nach außen zu verhindern. Die Seitenteile derartiger Inkontinenzwindeln sind vorzugsweise aus glatten Vliesmaterialien gebildet, um dort, wo keine sichere Flüssigkeitsbarriere erforderlich ist, die Hautfreundlichkeit der Windel zu verbessern. Somit würde eine Landefläche zum sicheren Festlegen der Kletthaken auf der Außenseite des Vorderbereichs der Windel das Anbringen eines zusätzlichen Materials, insbesondere eine an sich bekannte textile Loop-Komponente erfordern. Derartige Loop-Komponenten müssten sich allerdings über weite Teile des Vorderbereiches der Außenseite der Windel erstrecken, um das bei Inkontinenzwindeln (Windeln für Erwachsene) erforderliche hohe Maß an Flexibilität in der Größenanpassung zu gewährleisten. Da textile Loop-Komponenten einen wesentlichen Kostenfaktor bilden, verbietet sich eine derartige Lösung schon aus wirtschaftlichen Gründen.

Des Weiteren hat sich bei Inkontinenzwindeln der bekannten Art gezeigt, dass das subjektive Empfinden des Komforts der Windel trotz der hautfreundlichen und luftwie wasserdampfdurchlässigen Seitenteile deutliche Unterschiede ausweist.

Zur Lösung dieser Probleme wird eine Inkontinenzwindel mit den Merkmalen des Anspruch 1 vorgeschlagen, mithin dass die mechanische Verschlusshilfen aufweisenden Verschlussmittel zum bestimmungsgemäßen Festlegen der Windel an den Körper eines Menschen zumindest bereichsweise sowohl direkt an der Außenseite des Hauptteils als auch direkt an der Außenseite der Seitenteile im Vorderbereich lösbar festlegbar sind, wobei die Haltekräfte zwischen den Verschlussmitteln und der Außenseite des Hauptteils geringer sind als die Haltekräfte zwischen den Verschlussmitteln und der Außenseite der Seitenteile im Vorderbereich der Windel. Vorzugsweise werden die Haltekräfte als Über-Bauch-Haltekräfte ermittelt. Dadurch, dass die absorbierende Inkontinenzwindel vier diskrete, nicht direkt miteinander verbundene Seitenteile auf, derart, dass je ein Seitenteil an beiden Seitenrändern des Vorderbereichs und je ein weiteres Seitenteil an beiden Seitenrändern des Rückbereichs angefügt ist, kann der zwischen den vorderen und hinteren Seitenteilen liegende Schrittbereich der Windel seitenteilfrei belieben. Das kommt der Luftzirkulation der Windel im angelegten Zustand zugute. Zudem ist auf diese Art und Weise gleichsam produktionsabfallfrei (ohne Beinausschnitt) der Schrittbereich der Windel gebildet.

Die die Außenseite des Hauptteils und die die Außenseite der Seitenteile im Vorderbereich bildenden Materialien sind also erfindungsgemäß so gewählt, dass sie neben ihren jeweiligen Primärfunktionen auch als Landefläche für die mit mechanischen Verschlusshilfen versehenen Verschlussmittel dienen können. Überraschenderweise hat sich außerdem gezeigt, dass dann, wenn die Haltekräfte zwischen den Verschlussmitteln und der Außenseite des Hauptteils geringer sind als die Haltekräfte zwischen den Verschlussmitteln und der Außenseite der Seitenteile im Vorderbereich der Windel die Nutzer der Windel dazu tendieren, die Windel so zu schließen, dass die Verschlussmittel an der Außenseite der Seitenteile im Vorderbereich der Windel festgelegt werden. Dies wiederum erhöht den Tragekomfort, da hiermit ein Überlappen der Seitenteile mit dem Backsheet vermieden wird, wodurch erst das Wirksamwerden der Vorteile der Luft- und Wasserdampfdurchlässigkeit der Seitenteile ungehindert möglich wird. Außerdem ist die Gefahr des Beschädigens des Backsheets des Hauptteils und damit die Gefahr des Durchschlagens von Flüssigkeit durch die mechanischen Verschlusshilfen reduziert.

Wesentlich ist, dass auch die Haltekräfte zwischen der Außenseite des Hauptteils und den mechanische Verschlusshilfen aufweisenden Verschlussmitteln einen Halt der Windel am Körper gewähren. Hierzu hat es sich als vorteilhaft erwiesen, wenn die Über-Bauch-Haltekräfte zwischen den Verschlussmitteln und der Außenseite des Hauptteils 57-20 N/25mm, insbesondere 50-25 N/25mm betragen.

Des Weiteren betragen die Über-Bauch-Haltekräfte zwischen den Verschlussmitteln und der Außenseite der Seitenteile im Vorderbereich vorzugsweise 90-58 N/mm, insbesondere 80-60 N/25mm.

Zur Bestimmung der Über-Bauch-Haltekräfte werden die Verschlusskräfte bei Scherbeanspruchung gemessen. Nachfolgend wird eine Prüfmethode für die Ermittlung der Verschlusskräfte bei Scherbeanspruchung angegeben:

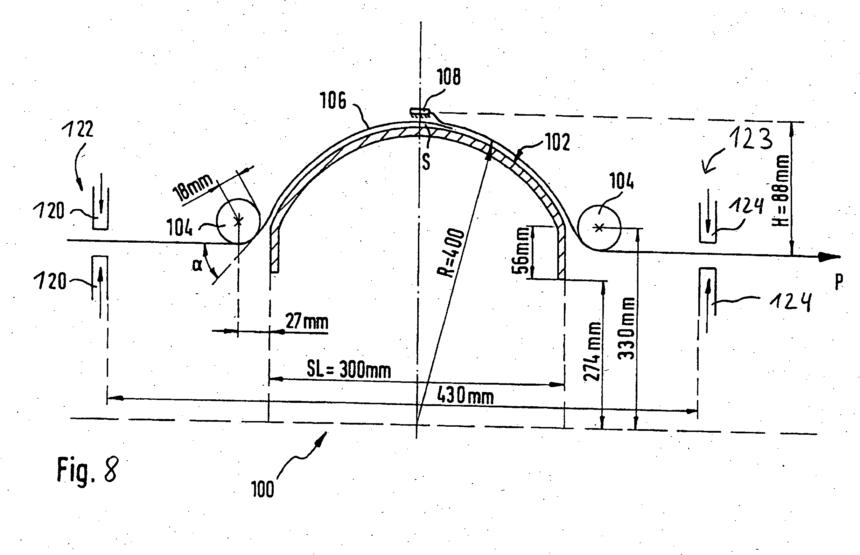

Für die Durchführung der Prüfmethode kann ein Zugprüfgerät Typ Z010 / TN 2S, Messdose 100 N, erhältlich bei der Firma Zwick GmbH & Co KG, Ulm, Deutschland, mit einer Klemmbackenbreite zum Einspannen des Prüflings von 60 mm verwendet werden. Bei der Durchführung der Prüfmethode wird das zu prüfende Verschlusssystem mit einer Schlaufen-Komponente und einem darauf haftenden mechanische Verschlusshilfen aufweisenden Verschlussmittel über eine gekrümmte Fläche gelegt, welche eine Rundung des Bauchbereichs eines Benutzers simulieren soll (siehe Figur 8). Zur Verbindung der Verschlusskomponenten mit den Klemmbacken des Zugprüfgeräts wird ein biegsames Substrat, beispielsweise ein einseitig klebendes Klebeband einer bevorzugten Breite von 25 mm, bzw. 50 mm, das unter der Bezeichnung STA 306 bei der Firma 3M Deutschland GmbH ansässig in Neuss erhältlich ist, verwendet. Das Klebeband ist aus Polypropylen, seine Oberfläche ist beschichtet durch ein urethanmodifiziertes Siliconpolymer. Das Flächengewicht des Haftklebstoffauftrags beträgt 23 g/m2. Der über die gekrümmte Fläche gelegte Prüfling, bestehend aus aufeinander haftenden flächenhaften Abschnitten des Verschlusssystems, wird durch Verwendung des Zugprüfgeräts auf Zug beansprucht, woraus sich eine scherende Beanspruchung der aneinander haftenden flächenhaften Abschnitte ergibt.

Probenvorbereitung:

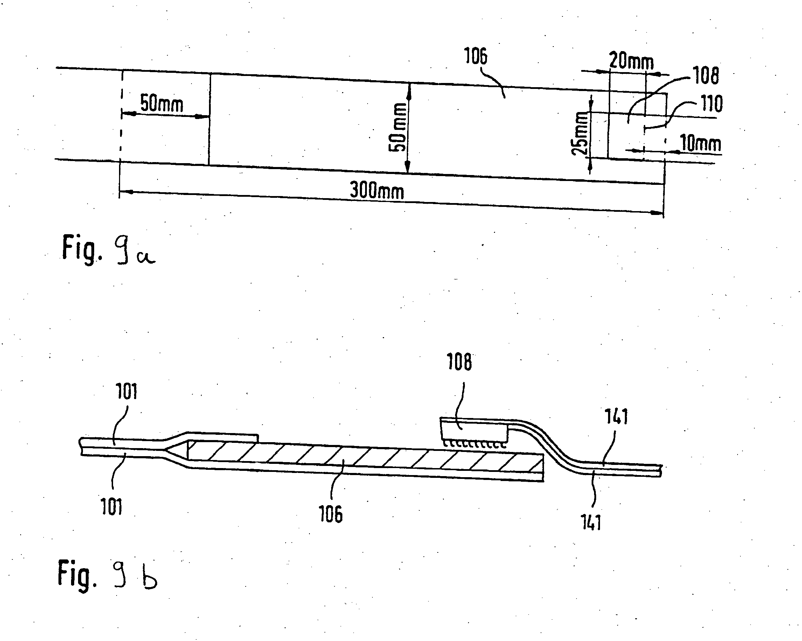

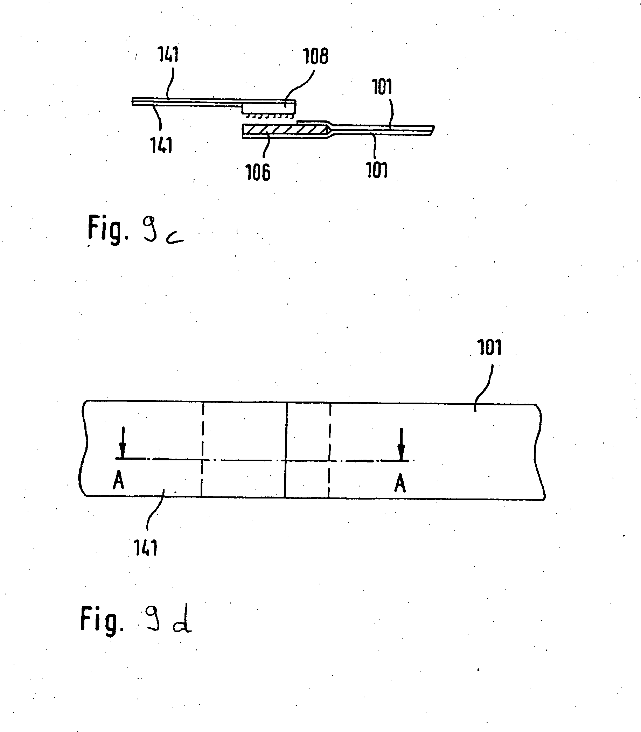

Die zu verwendenden mechanischen Verschlusskomponenten, also das Material der die Außenseite der vorderen Seitenteile oder des Hauptteils bildenden Schlaufen-Komponente 106 und ein mechanische Verschlusshilfen aufweisendes Verschlussmittel 108 des Verschlusssystems, werden über 24 h bei 23° C und 50 % relativer Luftfeuchte konditioniert. Wie unten noch näher beschrieben werden wird, kann es sich bei der Schlaufen-Komponente beispielsweise um einen Vliesstoff oder ein Vliesfolienlaminat handeln. Es werden Prüflinge einer Größe von 50 x 300 mm aus der Schlaufen-Komponente ausgestanzt und sandwichartig mittig durch zwei mit ihren Klebeflächen gegeneinander geklebte einseitige Klebebänder 101 einer Breite von 50 mm derart fixiert, dass das untere Klebeband die Rückseite des flächenhaften Abschnittes überfängt und das obere Klebeband die obere Seite (bei einem Vliesfolienlaminat ist die obere Seite die Vliesseite) der Schlaufen-Komponente in der Länge um 50 mm einfasst, so dass ein Überstand der Schlaufen-Komponente von 50 x 250 mm resultiert (siehe Figur 9a, Figur 9b) Desgleichen wird das mechanische Verschlusshilfen aufweisende Verschlussmittel 108 über seine volle Länge, das heißt der gleichen Länge, die bei der Inkontinenzwindel vorgesehen wird - im dargestellten Fall über 20 mm-, und in einer Breite von 25 mm ausgestanzt und durch zwei mit ihren Klebeflächen gegeneinander geklebte einseitige Klebebänder 141 einer Breite von 25 mm derart fixiert, dass das obere Klebeband die Rückseite des flächenhaften Abschnittes überfängt und das untere Klebeband bündig an den flächenhaften Abschnitt angrenzt (siehe Figur 9a und Figur 9b). Der flächenhafte Abschnitt der mechanische Verschlusshilfen aufweisenden Verschlussmittel 108 wird nun auf die Schlaufen-Komponente 106 aufgelegt, wobei der Abstand von der Längsendkante der Schlaufen-Komponente 10 mm und von den seitlichen Längsrändern je 12,5 mm betragen soll (s. Figur 9a). Wenn die zur Verfügung stehende Schlaufen-Komponente 106 von vornherein eine kleinere Abmessung aufweist, so dass keine Bereitstellung eines 50 mm x 300 mm großen Prüflings möglich ist, wird die Größe des Prüflings mit einer Breite von 25 mm und einer derart bemessenen Länge gewählt und dieser Abschnitt sandwichartig mittig zwischen die Enden zweier einseitiger oben näher gekennzeichneter Klebebänder 101 einer Breite von 25 mm derart fixiert, dass das untere Klebeband die Rückseite des flächenhaften Abschnittes überfängt und das obere Klebeband die obere Seite der Schlaufen-Komponente in der Länge derart einfasst, dass die Länge des Überstandes der Schlaufen-Komponente der Länge des Abschnittes des mechanischen Verschlusshilfen aufweisenden Verschlussmittels 108 entspricht (siehe Figur 9c, Figur 9d). Solchenfalls werden die so vorbereiteten Abschnitte des mechanische Verschlusshilfen aufweisenden Verschlussmittels 108 und der Schlaufen-Komponente 106 vollflächig übereinander gelegt (Figur 9c, Figur 9d).

Sollte kein 25 mm breiter Abschnitt des mechanische Verschlusshilfen aufweisenden Verschlussmittels 108 zur Verfügung stehen, wird ein entsprechend schmalerer Abschnitt verwendet. Solchenfalls werden die ermittelten Kraftwerte rechnerisch auf einen 25 mm breiten Abschnitt normiert, derart dass die gemessenen Kräfte mit einem Faktor f multipliziert werden, welcher sich ergibt aus $f = \dfrac{25}{x}$ , wobei x die Breite des Prüflings gemessen in mm ist.

Die so oder auf die zuvor beschriebene Art aufeinander gelegten flächenhaften Abschnitte werden durch viermaliges Anrollen mit einer 50 mm breiten und im Durchmesser 100 mm starken Rolle mit glatter Oberfläche und einem Rollengewicht von 5 kg miteinander verbunden, wobei die Anrollgeschwindigkeit 20-100 mm/sec beträgt.

Prüfverfahren:

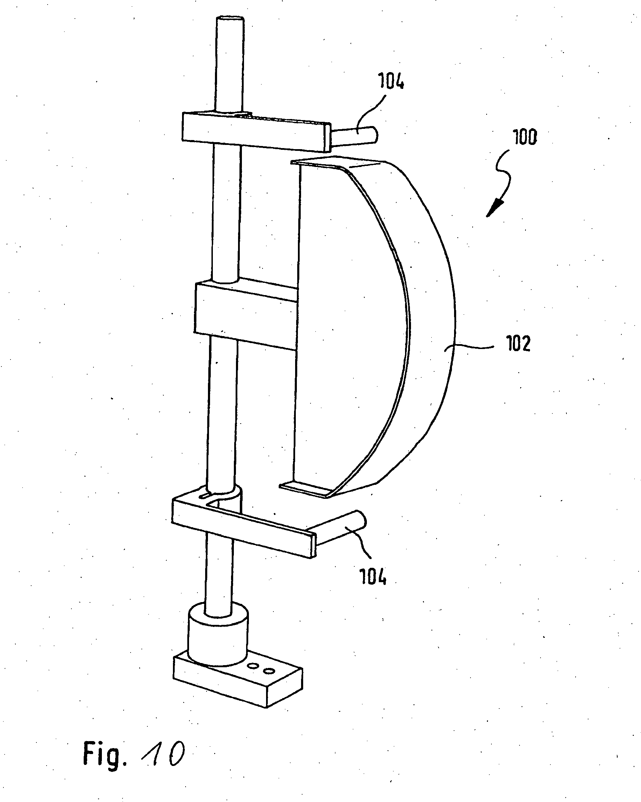

Die wie vorstehend beschrieben verlängerte Schlaufen-Komponente 106 wird in die untere Klemmbacke 122 des Zugprüfgeräts mittig zentriert eingespannt, und das gegenüberliegende Ende des wie vorstehend beschrieben verlängerten mechanische Verschlusshilfen aufweisenden Verschlussmittels 108 wird in die bewegliche obere Klemmbacke 123 des Zugprüfgeräts ebenfalls zentriert eingespannt. Der so eingespannte Prüfling wird über die aus Figur 8, Figur 10 ersichtliche Vorrichtung 100, welche den Bauch- oder Hüftbereich eines Benutzers simulieren soll, gelegt. Diese Vorrichtung 100 ist in Figur 10 perspektivisch dargestellt. Man erkennt eine bogenförmig gekrümmte Fläche 102 aus poliertem Stahl mit einer Rautiefe von 5 bis 25 mm und mit einem Krümmungsradius R von zumindest abschnittsweise 400 mm und einer Sehnenlänge SL von 300 mm. Des Weiteren sind oberhalb und unterhalb der gekrümmten Fläche 102 Umlenkrollen 104 mit einem Durchmesser von 18 mm vorgesehen, welche den über die gekrümmte Fläche gelegten Prüfling in die vertikale Richtung um H = 88 mm umlenken, wo er dann mit Klemmen 120, 124 des nicht dargestellten Zugprüfgeräts verbunden wird. Die Umlenkung erfolgt um einen Winkel $\alpha$ von 60°. Hierdurch wird der Abzugswinkel im Wesentlichen tangential zu der gekrümmten Fläche und konstant gehalten. Die aufeinander gelegten flächenhaften Abschnitte 106, 108 der Komponenten des Verschlussmittels werden in Bezug auf die gekrümmte Fläche 102 so positioniert, dass das mechanische Verschlusshilfen aufweisende Verschlussmittel mittig zentriert im Scheitelpunkt 5 der gekrümmten Fläche 102 zu liegen kommt. Es wird dann die bewegliche Klemmbacke 124, mit der das mechanische Verschlusshilfen aufweisende Verschlussmittel verbunden ist, mit der nachfolgend angegebenen Prüfgeschwindigkeit in Pfeilrichtung P bewegt, und es wird währenddessen die dabei auftretende Zugkraft zwischen den Klemmen ermittelt. Die Prüfparameter sind:

- Prüfgeschwindigkeit: 300 mm/min
- Einspannlänge des Prüflings: 430 mm (s. Figur 8)
- Messweg: Strecke bis zur Ablösung der Verschlussmittelkomponenten voneinander
- Vorkraft: 0,2 N
- Prüfanzahl: n ≥ 6.

Die Auswertung erfolgt dergestalt, dass die bis zum voneinander Ablösen der Verschlussmittel ermittelte Maximalkraft gerundet auf zwei Dezimalstellen in N (Newton) notiert wird und in Form eines Mittelwerts der n-Messungen angegeben wird.

Vorzugsweise ist jeweils ein die Kletthaken aufweisender Abschnitt in an sich bekannter Form auf einem jeweiligen Verschlussstreifenmaterial auflaminiert. Ein jeweiliger Verschlussstreifen ist vorzugsweise an einem freien Ende des jeweiligen Seitenteils verankert und weist seinerseits ein freies User-Ende mit den mechanischen Verschlusshilfen auf.

Es ist aber auch denkbar und vorteilhaft, auf jeden der Verschlussstreifen mehrere voneinander beabstandete Kletthaken aufweisende Abschnitte vorzusehen. Solchenfalls weist der Verschlussstreifen eine geringere Steifigkeit auf und kann sich der Rundung des Körpers im Tragezustand der Windel besser anpassen. Vorzugsweise weisen die einzelnen die Kletthaken aufweisenden Abschnitte eine Breite, das heißt eine Erstreckung in Querrichtung der Windeln von 1 - 10 mm, insbesondere 2-6 mm auf. Der Abstand zwischen den einzelnen Abschnitten beträgt insbesondere 0,1 - 3 mm, weiter insbesondere 0,7 - 2 mm.

Des Weiteren ist denkbar und vorteilhaft, das Verschlussstreifenmaterial wenigstens bereichsweise elastisch dehnbar auszubilden.

Des Weiteren ist denkbar und vorteilhaft, neben den mechanischen Verschlusshilfen, die als mechanische Elemente wie vorzugsweise Kletthaken ausgeführt sind, außerdem klebende Verschlusshilfen, wie haftklebende Bereiche auf dem Verschlussstreifen vorzusehen, um die Haftung zum Zwecke des Primärverschlusses oder sekundär zum Zwecke der Entsorgung der gebrauchten Windel noch sicherer zu gewährleisten. Die Haftklebezonen können insbesondere in einem äußeren, das heißt dem freien Ende eines Verschlussstreifens unmittelbar benachbarten Bereich eines jeweiligen Verschlussstreifens vorgesehen sein.

Des Weiteren ist denkbar, die die mechanischen Verschlusshilfen aufweisenden Verschlussmittel direkt auf der Innenseite der Seitenteile zu verankern. Solchenfalls wären die Verschlussmittel also nicht auf einem sich über das freie Ende des Seitenteils hinauserstreckenden Verschlussstreifen angeordnet, sondern innerhalb des Seitenrandes der Seitenteile.

Zumindest im Vorderbereich vorzugsweise auch im Rückbereich weisen die Seitenteile vorzugsweise eine Vlieskomponente auf, insbesondere sind die Seitenteile folienfrei ausgebildet, weiter insbesondere bestehen die Seitenteile aus einer ein- oder mehrlagigen Vlieskomponente.

Die Außenseite des Hauptteils der Inkontinenzwindel wird vorzugsweise zumindest bereichsweise, insbesondere aber vollflächig durch einen Vliesstoff gebildet. Vliesstoffe sind im Vergleich zu textilen Loop-Komponenten erheblich günstiger und außerdem als überaus hautfreundlich bekannt. Sie vermitteln der Inkontinenzwindel außerdem einen "textile-like" Eindruck. Solchenfalls ist es vorteilhaft, das Backsheet des Hauptteils aus einem Vliesfolienlaminat zu bilden, wobei die Vlieslage außen und die Folienlage innen zum Saugkörper gerichtet zu liegen kommt, so dass die Vlieslage die Außenseite des Hauptteils bildet. Damit ist zum einen die Flüssigkeitsundurchlässigkeit des Hauptteils sicher gestellt und zum anderen der hautfreundliche Charakter der Windel gesichert.

Die Folienlage dieses Vliesfolienlaminates ist vorzugsweise aus einer ein- oder mehrschichtigen flüssigkeitsundurchlässigen, vorzugsweise aber gleichwohl atmungsaktiven Folie gebildet. Damit ist der Vorteil einer Atmungsaktivität der Inkontinenzwindel auch im Bereich des Hauptteils erzielt.

Als Materialien für die Folie kommen im Prinzip alle thermoplastischen Polymere in Betracht. Hierzu ist eine Vielzahl von Handelsprodukten am Markt erhältlich. Vorzugsweise werden LDPE (Low Density Polyethylen), LLDPE (Linear Low Density Polyethylen), MDPE (Medium Density Polyethylen), HDPE (High Density Polyethylen) und verschiedene PP (Polypropylen) sowie Copolymerisate von Ethylen oder Propylen miteinander und mit anderen Comonomeren eingesetzt. Diese Polymere werden entweder in reiner Form oder als Polymergemische verwendet. Übliche Rezepturen für Hygienefolien sind z. B. Gemische aus 10 bis 90 Gew.-% LDPE, 10 bis 90 Gew.-% LLDPE und 0 bis 50% MDPE, zum Beispiel ein Gemisch aus 80% LDPE, 20% LLDPE und anforderungsgerechten Pigmentierungen.

Handelsübliche Polymere für Hygienefolien besitzen die nachfolgend angegebenen Schmelzbereiche bzw. Kristallitschmelzpunkte:

LDPE = 112 bis 114 °C,
LLDPE = 119 bis 125 °C
MDPE = 125 bis 128 °C.
PP (Blockcopolymere) = 130 bis 163°C

Weiterhin sind Ethylenvinylacetat (EVA), Ethylenacrylat (EEA), Ethylenethylacrylat (EEA), Ethylenacrylsäure (EAA), Ethylenmethylacrylat (EMA), Ethylenbutylacrylat (EBA), Polyester (PET), Polyamid (PA) z.B. Nylon, Ethylenvinylalkohole (EVOH), Polystyrol (PS), Polyurethan (PU) und thermoplastische Olefinelastomere als thermoplastische Polymermaterialien für die Folie geeignet.

Als Material der Folie sind Polyolefine wie z.B. LDPE, LLDPE und PP bevorzugt. Besonders bevorzugt sind Mischungen dieser Polymere, wie beispielsweise Mischungen aus LDPE und LLDPE, Mischungen aus LDPE oder LLDPE und PP oder Mischungen von PE oder PP verschiedener Schmelzpunkte.

Die Folie wird in an sich bekannter Weise hergestellt, z.B. durch Blasextrusion oder Cast-Verfahren. Bei diesen Verfahren während der Extrusion in die Folie eingebrachten Dehnungen sind wie weiter unten noch näher ausgeführt wird zumindest anteilig ursächlich dafür, dass die Folie bei einer späteren Temperung ein Schrumpfverhalten zeigt. Eine zusätzliche Reckung ist nicht erforderlich, kann aber gewünschtenfalls in an sich bekannter Weise erfolgen. Bei atmungsaktiven Laminaten erfolgt die Bereitstellung der Atmungsaktivität vorzugsweise durch Einmischen eines feinteiligen Füllstoffes und Reckung der Folie oder des Verbundes. Bei der Reckung entstehen Mikrorisse in der Folie, welche die geforderte Wasserdampf- und Gasdurchlässigkeit gewährleisten, ohne jedoch die Wasserdichtigkeit wesentlich zu beeinträchtigen.

Als Vliesstoffkomponenten für die Außenseite des Hauptteils und als Vliesstoffkomponente für die Seitenteile im Vorderbereich und vorzugsweise auch im Rückbereich kommt eine Vielzahl von Vliesstoffarten in Frage. Geeignet sind insbesondere alle Vliesstoffe, die zumindest eine Rezepturkomponente auf Basis eines thermoplastischen Polymers enthalten. Die Vliesstoffe können Fasern aus PE, PP, PET, Rayon, Zellulose, PA und Mischungen dieser Fasern enthalten. Auch Bi- oder Multikomponentenfasern sind denkbar und vorteilhaft. Vorteilhaft sind insbesondere Kardenvliese, Spinnvliese, wasserstrahlvernadelte Vliese, SM-Vliese, SMS-Vliese, SMMS-Vliese oder auch Laminate aus einer oder mehreren dieser Vliesarten, wobei S für Spunbond- und M für Meltblownvliesschichten steht. Besonders bevorzugt sind Spinnvliese, da diese eine hohe Festigkeit in Längs- und Querrichtung aufweisen und somit den auf sie durch das Eingreifen der mechanischen Verschlusshilfen einwirkenden Scherkräfte besonders gut standhalten können. Um zu verhindern, dass beim Lösen der mechanischen Verschlusshilfen Fasern aus dem Vliesverbund herausgerissen werden, ist vorteilhaft, die Vliesstoffkomponente mit einem Prägemuster zu versehen, vermittels dessen vorzugsweise alle Fasern der Vlieskomponente gebunden sind. Vorteilhaft ist insbesondere ein Thermoprägemuster, das insbesondere vorteilhaft durch Kalandern des Vliesstoffes unter Zuführung von thermischer Energie erzeugt wird.

Das Prägemuster kann in an sich bekannter Weise eine Vielzahl von punktförmigen Fügestellen oder Fügebereichen umfassen, wobei die Fügestellen jede denkbare geometrische Form annehmen kann. Insbesondere können die Fügestellen kreisförmig, oval, quadratisch, rechteckig, rautenförmig oder sternförmig sein.

Nach einer besonders vorteilhaften Ausführungsform sind die Fügebereiche des Prägemusters hingegen so angeordnet, dass die Fügebereiche nicht gebundene inselartig angeordnete Schlaufenbereiche umgeben. Die Fügebereiche können die Schlaufenbereiche kontinuierlich, gleichsam lückenlos umgeben. Denkbar und vorteilhaft ist jedoch auch, die Fügebereiche aus einer Vielzahl von insbesondere kleineren Abschnitten linienförmiger Fügestellen zu bilden, die Fügebereiche also mit Durchbrechungen zu versehen.

Die geometrische Form der nicht gebundenen inselartig angeordneten Schlaufenbereiche ist an sich unkritisch. Die Schlaufenbereiche können kreisförmig, oval, rechteckig, quadratisch, dreieckig, sechseckig, achteckig oder andere mehreckige Formen annehmen.

Insbesondere können die Fügebereiche durch eine Vielzahl von Linien gebildet sein, die sich zur Ausbildung eines regelmäßigen Rautenmusters schneiden, so dass inselartig angeordnete unverbundene, rautenförmige Schlaufenbereiche von linienartigen Fügebereichen umgeben sind. Die die mechanischen Verschlusshilfen aufweisenden Verschlussmittel sind mit diesen Schlaufenbereichen sicher in Eingriff bringbar, ohne dass die Gefahr besteht, Fasern aus der Vlieskomponente herauszulösen. Ein derartiges Prägemuster ist beispielsweise in EP0882828A1 offenbart.

Ein weiteres bevorzugtes Prägemuster mit inselartig angeordneten Schlaufenbereichen ist in DE102004053469A1 offenbart, worauf hiermit ausdrücklich Bezug genommen wird. Dieses Prägemuster offenbart bereits die zuvor erwähnten mit Durchbrechungen versehenen Fügebereiche.

Der Flächenanteil der Fügestellen oder Fügebereiche beträgt vorzugsweise 7-40%, insbesondere 15-30 %, weiter insbesondere 17-25%.

Das Flächengewicht der Vliesstoffkomponente für die Außenseite des Hauptteils beträgt vorzugsweise 10-30 g/m$^2$, insbesondere 14-25 g/m$^2$, weiter insbesondere 18-22 g/m$^2$.

Das Flächengewicht der Vliesstoffkomponente für die Seitenteile beträgt vorzugsweise 18-60 g/m$^2$, insbesondere 25-45 g/m$^2$, weiter insbesondere 27-40 g/m$^2$ und weiter insbesondere 28-35 g/m$^2$.

Vorzugsweise ist das Flächengewicht der Vliesstoffkomponente für die Seitenteile im Vorderbereich und vorzugsweise auch im Rückbereich größer als das Flächengewicht der Vliesstoffkomponente für die Außenseite des Hauptteils. Durch das höhere Flächengewicht der Vliesstoffkomponente für die Seitenteile ist eine höhere Verschlusshaltekraft möglich, da die mechanischen Verschlusshilfen mit mehr Fasermaterial in Eingriff bringbar sind. Das höhere Flächengewicht der Vliesstoffkomponente der Seitenteile veranlasst außerdem den Benutzer, die mechanische Verschlusshilfen aufweisenden Verschlussmittel bevorzugt an den Seitenteilen und nicht an der Außenseite des Hauptteils zu fixieren, da der Benutzer mit einem höheren Flächengewicht und damit vorzugsweise mit einer höheren Dicke der Vliesstoffkomponente der Seitenteile eine höhere Verschlusssicherheit assoziiert. Vorzugsweise weist daher die Vliesstoffkomponente der Seitenteile eine höhere Dicke als die Vliesstoffkomponente der Außenseite des Hauptteils auf, wobei die Dicke unter einem Prüfdruck von 0,5 kPa ermittelt wird.

In einer besonders bevorzugten Ausführungsform weisen die Vliesstoffkomponenten der Außenseite des Hauptteils und der Seitenteile dasselbe Prägemuster auf. Dies ist der Optik des Hygieneartikels und dem subjektiven Komfortempfinden des Anwenders zuträglich.

Die Vliesstoffkomponente der Außenseite der Seitenteile im Vorderbereich und vorzugsweise auch im Rückbereich ist vorzugsweise aus einem für wässrige Flüssigkeiten durchlässigen Material gebildet. Dies fördert die Abführung von Schweiß von innen nach außen.

In einer besonders bevorzugten Ausführungsform bestehen die Seitenteile im Vorderbereich und vorzugsweise auch im Rückbereich im Wesentlichen aus einer Vliesstoffkomponente, so dass sowohl die Außenseite als auch die Innenseite der Seitenteile im Wesentlichen durch die Vliesstoffkomponente gebildet werden.

Die Außenseite eines das Backsheet einer erfindungsgemäßen Inkontinenzwindel bildenden Vliesfolienlaminates soll mit den die mechanischen Verschlusshilfen aufweisenden Verschlussmitteln eine Über-Bauch-Haltekraft gewährleisten, die einen sicheren Halt der Windel am Körper sicher stellt. Hierzu ist vorteilhaft, neben der geeigneten Auswahl der Vliesstoff- und der Folienkomponente, ein geeignetes Laminierverfahren zur Herstellung des Verbundes aus Vlies und Folie anzuwenden.

Das Laminieren der Vliesstoffkomponente des Backsheets mit der Folienkomponente des Backsheets kann in an sich bekannter Weise durch beliebige Fügeverfahren insbesondere durch Verkleben, Verprägen, Ultraschallschweißen oder thermisches Kalandrieren erfolgen. Ein bevorzugtes Thermolaminierverfahren ist in DE102004042405A1 offenbart. Auf die DE102004042405A1 wird in Bezug auf das Thermolaminierverfahren ausdrücklich Bezug genommen und dieses Verfahren insoweit auch zur Offenbarung dieser nun vorgelegten Beschreibung gehörig erklärt. Es ist auch möglich, das Laminat durch Direktextrusion der Folie auf das Vlies herzustellen. Solche Verbunde werden vorzugsweise einem Reckschritt in zumindest einer Richtung unterworfen, um den erreichbaren Schrumpf bei der - unten näher beschriebenen - Heißtemperung zu vergrößern. Laminate, bei welchen die Folie durch Direktextrusion mit der Vlieslage verbunden wurde, werden daher geprägt, gereckt, insbesondere durch an sich bekanntes Ring-Rolling, oder vorzugsweise geprägt und gereckt.

Da eine Vielzahl der Laminierverfahren zur Herstellung des Vliesfolienlaminates eine Beeinträchtigung der Bauschigkeit der Vliesstoffkomponente und damit eine deutliche Reduzierung der Über-Bauch-Haltekräfte nach sich ziehen, hat es sich als vorteilhaft erwiesen, das Vliesfolienlaminat nach der Laminierung einer Temperung zuzuführen. Diese Temperung hat zur Folge, dass die Folie schrumpft, insbesondere um 1-10 %, weiter insbesondere um 2-5 %, insbesondere in der Richtung in der sie vormals im Zuge ihrer Herstellung oder während der Laminierung gestreckt wurde, das heißt in Quer- und/oder in Längsrichtung. Die Schrumpfung der Folie zwingt die mit ihr verbundene Vliesstoffkomponente zur einer Art Raffung, mit der auch eine Flächengewichtszunahme einhergeht, so dass die Vliesstoffkomponente nachfolgend besser mit den mechanische Verschlusshilfen aufweisenden Verschlussmitteln in Eingriff bringbar ist und höhere Über-Bauch-Haltekräfte ausweist als vor der Temperung. Vorzugsweise erfolgt die Temperung als Heißtemperung oberhalb der Schmelztemperatur der Folien. Vorzugsweise weist die Folie solchenfalls zumindest einen Bestandteil auf, der einen geringeren Schmelzpunkt besitzt als zumindest eine Faserkomponente der Vliesstoffkomponente, so dass die Faserstruktur durch das Heißtempern nicht zerstört wird.

Unter Schmelzpunkt wird im Rahmen der vorliegenden Anmeldung im Hinblick auf die polymeren Materialien diejenige Temperatur verstanden, bei welcher der Schubmodul des Materials gegen null geht. Soweit es sich um Polymere mit kristallinen Anteilen oder um kristalline Polymere handelt, sind bei dieser Temperatur (auch) die kristallinen Bereiche geschmolzen.

In Bezug auf eine Folie ist der Schmelzpunkt diejenige Temperatur, bei der die Folie insgesamt aufschmilzt. Sofern die Folie nicht nur aus einem Material besteht, kommt es nicht darauf an, dass sämtliche Komponenten für sich eine Schmelztemperatur bei oder unterhalb des Schmelzpunktes aufweisen, vielmehr entspricht der Schmelzpunkt der Folie regelmäßig der Schmelztemperatur der thermoplastischen Hauptkomponente. So weist eine atmungsaktive Folie enthaltend 60 % Calciumcarbonat, 32 % eines Polymers mit einer Kristallitschmelztemperatur von 138 °C und 8 % eines Polymers mit einer Kristallitschmelztemperatur von 158 °C einen Schmelzpunkt von etwa 138 °C auf.

Bei atmungsaktiven Folien wird die Atmungsaktivität z.B. überwiegend durch Zusatz eines feinteiligen Füllstoffs wie Calciumcarbonat erreicht, dessen sehr hohe Schmelztemperatur aber bei Zusätzen selbst von über 50 Gew.-% den Schmelzpunkt der Folie nicht wesentlich beeinflusst. So weist eine Folie aus einem Gemisch von 60 % Calciumcarbonat mit 40 % Polymer denselben Kristallitschmelzpunkt wie das Polymer auf.

Bei Vliesen brauchen im Falle von mehreren Materialkomponenten nicht alle Materialkomponenten einen Schmelzpunkt oberhalb der Temperatur des Heißtemperns zu haben, es reicht, wenn zumindest eine Komponente einen höheren Schmelzpunkt hat, so dass die Integrität des Vlieses gewahrt bleibt. Es kann sogar vorteilhaft sein, wenn eine Materialkomponente des Vlieses eine geringere Schmelztemperatur aufweist, da hierdurch die Verbundhaftung der Laminate verbessert werden kann.

Die bevorzugte Heißtemperung des Vliesfolienlaminates kann entweder direkt im Anschluss an seine Herstellung (in-line) oder unabhängig davon zu einem späteren Zeitpunkt (off-line) erfolgen. Die Heißtemperung erfolgt auf an sich für die Temperung bekannte Weise, jedoch ist die Temperatur zumindest oberhalb der Schmelztemperatur der thermoplastischen Hauptkomponente der Folie des Vliesfolienlaminates einzustellen.

Das Prozessfenster der Erwärmung ist bei der minimalen Temperatur durch den im Falle der Heißtemperung zwingend erforderlichen schmelzeflüssigen Zustand der Folie gegeben. Nach oben wird die Erwärmung durch den Kristallitschmelzpunkt der Vliesbahn begrenzt, sowie bei atmungsaktiven Folien gegebenenfalls zusätzlich durch einen bei zu hohen Temperaturen auftretenden Verlust der Atmungsaktivität der Folie. Wird in den Kristallitschmelzpunkt der Vliesbahn hinein erwärmt, geht die gute Weichheit des Laminates verloren und es entsteht die Gefahr der Bildung von Löchern (pin-holes) in dem Verbund. Bei atmungsaktiven Folien sollte die Heißtemperung bei solchen Temperaturen erfolgen, dass die Schmelze noch zu zähflüssig ist, um die Poren zu verschließen. Alternativ kann aber die Atmungsaktivität auch erst nach dem Heißtempern erzeugt werden, indem der Verbund nach diesem Schritt z.B. noch einem Ring-Rolling unterworfen wird.

Die Heißtemperung erfolgt vorzugsweise, indem das Vliesfolienlaminat mittels einer oder mehrerer Heizwalzen oder alternativ z.B. mittels IR-Strahlung soweit erwärmt wird, dass die Folie, d.h. zumindest die niedrigschmelzende Rohstoffkomponente der Folie, den schmelzeflüssigen Zustand erreicht. Nach erfolgter Erwärmung läuft der Verbund direkt auf langsamer laufende Kühlwalzen, so dass eine schockartige Kühlung auftritt und durch die 1 - 10 % geringere Umfangsgeschwindigkeit ein Schrumpf zwischen Heizelement und Kühlung möglich ist. Der Schrumpf sollte im Bereich von 1 - 10 % (Länge/Länge) in Maschinenrichtung liegen, quer dazu erfolgt je nach Herstellung und Vorbehandlung der Folie ebenfalls ein Schrumpfen. Bei Blasfolien liegt der Querschrumpf typischerweise ebenfalls bei 1 bis 10 %, bei Cast-Folien ist er typischerweise geringer. Die Temperatur der Kühlwalzen beträgt vorzugsweise 10 - 30°C, so dass die Kühlung des Verbundes auf unterhalb der Schmelztemperatur der Folie sehr schnell, z.B. innerhalb von Bruchteilen einer Sekunde erfolgt.

Es hat sich des Weiteren als vorteilhaft erwiesen, wenn die Atmungsaktivität der Seitenteile im Vorderbereich und vorzugsweise auch im Rückbereich größer ist als die Atmungsaktivität des Backsheets.

In vorteilhafter Weiterbildung der Erfindung sind die Seitenteile im Vorder- und/oder Rückbereich wenigstens um eine in Längsrichtung verlaufende Falzlinie auf sich selbst gefaltet. Insbesondere sind hierbei aufeinander gefaltete und flächenhaft aneinander liegende Teilabschnitte der Seitenteile in dieser gefalteten Konfiguration an Fügestellen oder Fügebereichen lösbar aneinander fixiert. Diese lösbare Fixierung ist vorzugsweise durch thermisch oder durch Ultraschall erzeugte, vorzugsweise punktförmige Fügestellen ausgebildet. Dies hat den Vorteil, dass die Seitenteile in der schnell laufenden Herstellungsmaschine sicher geführt werden können und auch beim Entnehmen aus einer Umverpackung und beim Vorbereiten zum Anlegen der Inkontinenzwindel Vorteile resultieren. Hierdurch wird die Inkontinenzwindel bedienungsfreundlich und erweist sich insbesondere bei der Anwendung bei starken pflegebedürftigen Personen besonders vorteilhaft.

In Weiterbildung dieses Erfindungsgedankens erweist es sich als vorteilhaft, wenn in einem in Querrichtung das freie Ende des Seitenteils bildenden Teilabschnitt eines jeweiligen so gefalteten Seitenteils ein Anfassbereich zum Entfalten des Materialabschnitts vorgesehen ist. Dieser Anfassbereich kann im einfachsten Fall von einem Längsseitenrandabschnitt des erwähnten Teilabschnitts gebildet sein, der mit den Fingern eines Benutzers ergreifbar ist. Es wäre auch denkbar, dass an dem betreffenden Teilabschnitt ein separates manuell ergreifbares Anfasselement vorgesehen ist, was jedoch einen zusätzlichen herstellungstechnischen Aufwand bedeuten würde.

Es erweist sich als besonders vorteilhaft, wenn die lösbare Fixierung an sämtlichen Fügestellen oder Fügebereichen beim Entfalten durch einmaliges Ziehen an dem Anfassbereich der jeweiligen Seitenteile auftrennbar ist. Hierdurch wird die Handhabung weiter vereinfacht und die Inkontinenzwindel wird hierdurch noch bedienungsfreundlicher. In Bezug auf die Bedeutung und die Prüfung des Merkmals des Entfaltens durch einmaliges Ziehen wird ausdrücklich auf die DE102004021353A1 und die dort offenbarte Prüfmethode Bezug genommen.

In Weiterbildung dieses Erfindungsgedankens sind die Seitenteile im Vorderbereich und vorzugsweise auch im Rückbereich um wenigstens zwei Falzlinien auf sich selbst gefaltet, so dass eine im Schnitt z-förmige Konfiguration entsteht. Nach einer weiteren bevorzugten Ausführungsform sind die Seitenteile um drei Falzlinien auf sich selbst gefaltet.

Nach einer weiteren bevorzugten Ausführungsform der erfindungsgemäßen Inkontinenzwindel sind die jeweiligen Anfassbereiche vor der Entfaltung der Seitenteile im Vorderbereich und vorzugsweise auch im Rückbereich in Querrichtung nach außen gewandt, also voneinander und von einer Längsmittelachse des auf einer ebenen Unterlage ausgebreiteten Windelhauptteils voneinander weg gewandt, so dass sie auf bequeme Weise mit der linken Hand eines Benutzers von links und mit der rechten Hand von rechts ergreifbar sind.

Die Erstreckung eines an den Hauptteil angefügten Seitenteils im entfalteten Zustand in Querrichtung über den Seitenrand des Hauptteils hinaus, beträgt vorzugsweise wenigstens 10 cm, insbesondere wenigstens 15 cm, und weiter insbesondere wenigstens 18 cm. Sie beträgt vorzugsweise höchstens 35 cm, insbesondere höchstens 30 cm und weiter insbesondere höchstens 27 cm. Die an den Hauptteil angefügten Seitenteile haben im Bereich der Anfügung an den Hauptteil eine Erstreckung in Längsrichtung der Inkontinenzwindel von vorzugsweise wenigstens 10 cm, insbesondere wenigstens 14 cm, insbesondere wenigstens 18 cm und weiter insbesondere wenigstens 22 cm.

In Weiterbildung der Erfindung weisen die Seitenteile zumindest im Rückbereich ein Verstärkungsmittel auf, welches in Querrichtung betrachtet schmäler ausgebildet ist als ein jeweiliges Seitenteil und welches wenigstens in einem den Seitenrand des Hauptteils überbrückenden Bereich vorgesehen ist, also sowohl einen Seitenrandbereich des Hauptteils als auch einen Teil des Seitenteils in Querrichtung überfängt. Auf diese Weise wurde die Einreißfestigkeit der Seitenteile in beträchtlichem Maße erhöht.

Es erweist sich als besonders vorteilhaft, wenn das Verstärkungsmittel im Wesentlichen wenigstens nahezu bis zu einem dem Schrittbereich zugewandten Querrand des Seitenteils erstreckt ist, wenn es also vorzugsweise kantenbündig mit dem Querrand des Seitenteils abschließt oder gar den Querrand einschließt oder umschließt.

Das Verstärkungsmittel könnte in der Längsrichtung des Hygieneartikels, beispielsweise über die gesamte Längsausdehnung des angefügten Seitenteils erstreckt sein. Es hat sich indessen gezeigt, dass dies nicht zwingend erforderlich ist, sondern dass es sich als ebenfalls vorteilhaft erweist, wenn das Verstärkungsmittel in Längsrichtung des Hygieneartikels eine geringere Abmessung aufweist als das angefügte Seitenteil selbst. Infolge der beim Gebrauch auftretenden Krafteinwirkung auf das Seitenteils und auf den Anfügungsbereich von Seitenteil und Windelhauptteil ist es durchaus hinreichend, wenn das Verstärkungsmittel sich beispielsweise nur bis 80 % oder insbesondere bis 60 % und weiter insbesondere bis 50 % der Längserstreckung des Seitenteils erstreckt. Hierdurch kann gegenüber der in Längsrichtung durchgehenden Verstärkung eine Materialersparnis erzielt werden.

Das Verstärkungsmittel erstreckt sich stets in Querrichtung über den Seitenrand des Hauptteils hinaus in Richtung des freien Endes des Seitenteils. Diese Erstreckung des in Richtung des freien Endes des Seitenteils über den Seitenrand des Hauptteils hinausgehenden Bereichs des Verstärkungsmittels, gemessen von Seitenrand des Hauptteils, beträgt vorzugsweise höchstens 50%, insbesondere höchstens 35%, weiter insbesondere höchstens 25%, weiter insbesondere höchstens 20%, weiter insbesondere höchstens 15%, weiter insbesondere höchstens 10% der Quererstreckung des Seitenteils.

Vorzugsweise erstreckt sich das Verstärkungsmittel außerdem in Querrichtung in Richtung der Längsmittellinie des Hauptteils, so dass es den Überlappungsbereich von Seitenteil und den Materialien des Hauptteils zumindest teilweise überfängt. Vorzugsweise überfängt das Verstärkungsmittel den Überlappungsbereich vollständig.

Als ganz besonders vorteilhaft erweist es sich, dass ein jeweiliges angefügtes Seitenteil rechteckförmig ausgebildet werden kann, ohne dass die erwähnte Einreißproblematik dem entgegenstehen würde.

Das erfindungsgemäß vorgesehene Verstärkungsmittel kann nach einer ersten Ausführungsform der Erfindung in vorteilhafter Weise von einem angefügten Verstärkungsabschnitt gebildet sein, also von einem zusätzlichen dem jeweiligen Seitenteil zugefügten, insbesondere auf den jeweiligen Seitenteil aufgebrachten Material. Dieser Verstärkungsabschnitt kann beispielsweise streifenförmig ausgebildet sein. Dieser Verstärkungsabschnitt kann weiterhin jegliche Form annehmen. Dieser Verstärkungsabschnitt kann beispielsweise auch in Form eines Dreiecks ausgebildet sein.

Es kann sich hierbei um einen Abschnitt eines streifen- oder bandförmigen Materials handeln. Insbesondere und vorteilhafterweise kann der Verstärkungsabschnitt von einem Vliesmaterial, einem textilen Material oder einer Folie gebildet sein. Er kann ebenso wie die Seitenteile in einem endlosen Fertigungsprozess im Cut & Place-Verfahren zugeführt und angefügt werden.

Im Falle der Ausbildung des Verstärkungsabschnitts in Form eines Vliesstoffes und der Anordnung des Verstärkungsabschnittes auf der Außenseite der Seitenteile ist die Außenseite des Vliesstoffes vorzugsweise mit den mechanische Verschlusshilfen aufweisenden Verschlussmitteln, etwa zum Zwecke der Entsorgung der gebrauchten Windel, in Eingriff bringbar.

Vorzugsweise weist der Verstärkungsabschnitt dasselbe Prägemuster auf wie die Vliesstoffkomponente des Seitenteils und/oder der Außenseite des Hauptteils.

Es können auch mehrere Verstärkungsabschnitte vorgesehen sein. Die Verstärkungsmittel können an eine oder beide Oberseiten des Seitenteils angefügt sein.

Nach einer besonders vorteilhaften Ausführungsform der Erfindung ist das Verstärkungsmittel von dem Material des jeweiligen Seitenteils selbst gebildet, indem das Seitenteil in dem den Seitenrand des Hauptteils überbrückenden Bereich einmal oder mehrmals gefaltet ist. In der Draufsicht auf den eben ausgefalteten Hygieneartikel ist nach dieser Ausführungsform also ein den Seitenrand des Hauptteils überfangender oder überlappender Bereich des jeweiligen Seitenteils durch eine Materialverdoppelung oder -vervielfachung durch Falten des Seitenteils gebildet. Hierdurch kann ein besonders effektiver Einreißschutz geschaffen werden. Als besonders vorteilhaft erweist sich eine in der Längsrichtung des Hygieneartikels betrachtete Z-förmige Faltung des jeweiligen Seitenteils.

Weitere Merkmale, Einzelheiten und Vorteile der Erfindung ergeben sich aus den beigefügten Patentansprüchen und aus der zeichnerischen Darstellung und nachfolgender Beschreibung. Bevorzugte Ausführungsformen der erfindungsgemäßen Windel in der Zeichnung zeigen:

Figur 1 eine Draufsicht auf die Außenseite einer erfindungsgemäßen Inkontinenzwindel

Figur 2 eine Querschnittsansicht entlang einer Linie A-A der in Figur 1 dargestellten Inkontinenzwindel

Figur 3 ein Prägemuster für eine Vliesstoffkomponente

Figur 4 ein weiteres Prägemuster für eine Vliesstoffkomponente

Figur 5 eine schematische Darstellung der Anfügung eines Seitenteils an einen Hauptteil mit Verstärkungsmittel

Figuren 6 und 7 Darstellung eines Kletthakens einer mechanischen Verschlusshilfe

Figur 8 eine schematische Darstellung des Aufbaus eines Zugprüfversuches mit einer Vorrichtung mit gekrümmter Fläche

Figuren 9a-9d schematische Darstellungen des Prüflings

Figur 10 eine perspektivische Ansicht der in Figur 8 dargestellten Vorrichtung

Figuren 11a, 11 b Draufsichten auf das User-Ende von Verschlussstreifen einer Inkontinenzwindel

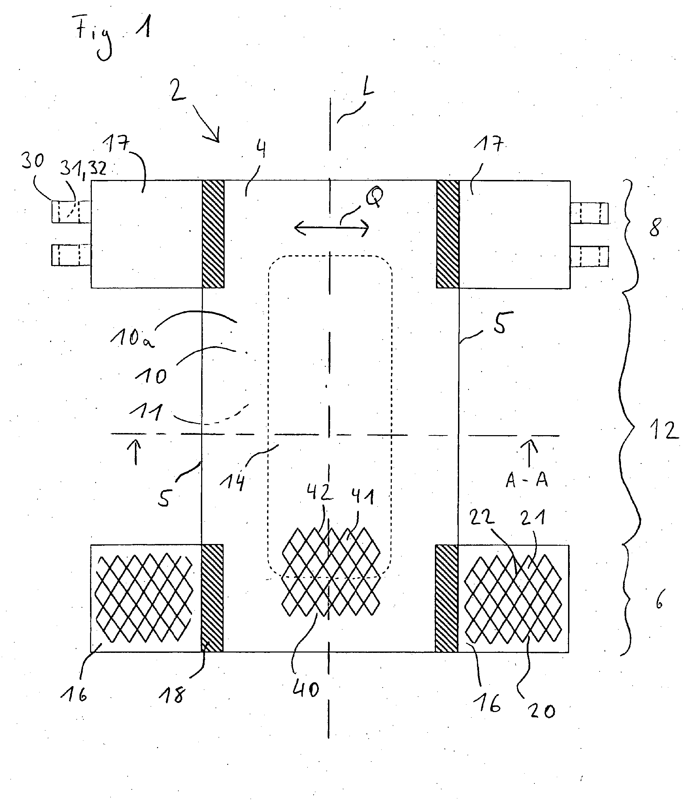

Figur 1 zeigt schematisch eine Draufsicht auf die Außenseite einer absorbierenden Inkontinenzwindel 2 in eben ausgefaltetem Zustand. Die Inkontinenzwindel umfasst einen Hauptteil 4 mit einer Längsmittellinie L bestehend aus einem Vorderbereich 6, einem Rückbereich 8 und einem in Längsrichtung L dazwischen liegenden Schrittbereich 12. Außerdem angedeutet ist ein Saugkörper 14, der üblicherweise zwischen chassisbildenden Materialien des Hauptteils, also insbesondere zwischen einem flüssigkeitsdurchlässigen Topsheet 11 und einem im Wesentlichen flüssigkeitsundurchlässigen Backsheet 10 des Hauptteils 4 angeordnet ist. Es sind aber auch Ausführungsformen denkbar, bei denen der Saugkörper als separate mit Auslaufschutz versehene Einheit auf eine chassisbildende Lage des Hauptteils aufbringbar und dort lösbar oder unlösbar fixierbar ist.

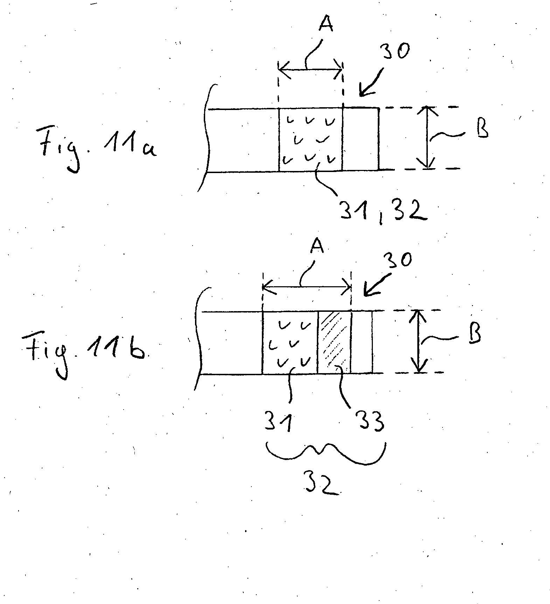

Die Inkontinenzwindel 2 umfasst des Weiteren vordere Seitenteile 16 und hintere Seitenteile 17, die als separate Vliesstoffkomponenten beidseits an den Hauptteil 4 angefügt sind. Sie haben jeweils rechteckförmige Gestalt, was nicht zwingend, jedoch im Hinblick auf die Vermeidung von Schnittabfall vorteilhaft ist. Die Seitenteile sind in einem schraffiert dargestellten Überlappungsbereich 18 mit chassisbildenden Materialien des Hauptteils 4, also beispielsweise mit dem Backsheet 10 und/oder dem Topsheet 11 zum bestimmungsgemäßen Gebrauch unlösbar verbunden. Sie erstrecken sich über seitliche Längsränder 5 des Hauptteils 4 in Querrichtung Q des Hauptteils 4 hinaus. Die Seitenteile 16, 17 sind dafür gedacht und bestimmt, im angelegten Zustand der Inkontinenzwindel miteinander verbunden zu werden, um einen in Umfangsrichtung durchgehenden Hüftbereich des Hygieneartikels zu bilden. Dabei werden jeweils die auf einer Seite des Hauptteils vorgesehenen Seitenteile 16, 17 miteinander verbunden. Hierzu sind die mechanische Verschlusshilfen 31, insbesondere Kletthaken, aufweisenden Verschlussmittel 32 der Verschlussstreifen 30 auf der Außenseite der vorderen Seitenteile 16 festlegbar. Figur 11a zeigt in der Draufsicht in vergrößerter Darstellung das User-Ende einer der Verschlussstreifen 30 mit einem Verschlussmittel 32, das allein durch den Abschnitt mechanischer Verschlusshilfen 31 in Form von Kletthaken gebildet ist. Die Breite B des Abschnittes mechanischer Verschlusshilfen 31 beträgt vorliegend 25mm und dessen Länge A beträgt vorliegend 20mm. Figur 11b zeigt eine alternative Ausführungsform des User-Endes eines Verschlussstreifens 30 mit einem Verschlussmittel 32, das aus einem Abschnitt mechanischer Verschlusshilfen 31 und einem Abschnitt klebender Verschlusshilfen in Form eines haftklebenden Bereiches 33 gebildet ist, wobei die Breite B des Verschlussmittels 32 25mm beträgt und die Länge A des Verschlussmittels 32 30mm beträgt.

Zumindest die vorderen Seitenteile 16, vorzugsweise auch die hinteren Seitenteile 17 sind aus einer Vliesstoffkomponente, im dargestellten Falle aus einem PP-Spinnvlies von 30 g/m² gebildet. Die Faserstärke beträgt 2 dtex. Die Außenseite des Spinnvliesstoffes weist ein in Figur 1 nur schematisch angedeutetes Prägemuster 20 auf. Die durch Heißkalanderprägung erzeugten Fügebereiche sind durch eine Vielzahl von Linien gebildet, nämlich durch zwei Gruppen von innerhalb einer Gruppe jeweils parallel verlaufenden Linien, wobei sich die Linien der einen mit der der anderen Gruppe zur Ausbildung eines regelmäßigen Rautenmusters unter einem Winkel von 33° schneiden, so dass inselartig angeordnete unverbundene, rautenförmige Schlaufenbereiche 21 von linienartigen Fügebereichen 22 umgeben sind. Die Fügebereiche 22 bildenden Linien haben im dargestellten Fall eine Breite von 1,0 mm und eine Prägetiefe von 0,6 mm. Der Abstand zweier benachbarter parallel verlaufender Linien beider Gruppen von Linien beträgt 4,7 mm. Die Prägefläche, also die Summe der Fläche aller Fügebereiche 22 bezogen auf die Gesamtfläche des Prägemusters (Fügebereiche + Schlaufenbereiche) beträgt 32 %.

Die auf der Innenseite der Verschlussstreifen 30 der hinteren Seitenteile 17 angeordneten Verschlussmittel 32 sind mit diesen Schlaufenbereichen 21 sicher in Eingriff bringbar, ohne dass die Gefahr besteht, Fasern im Übermaß aus der Vliesstoffkomponente herauszulösen.

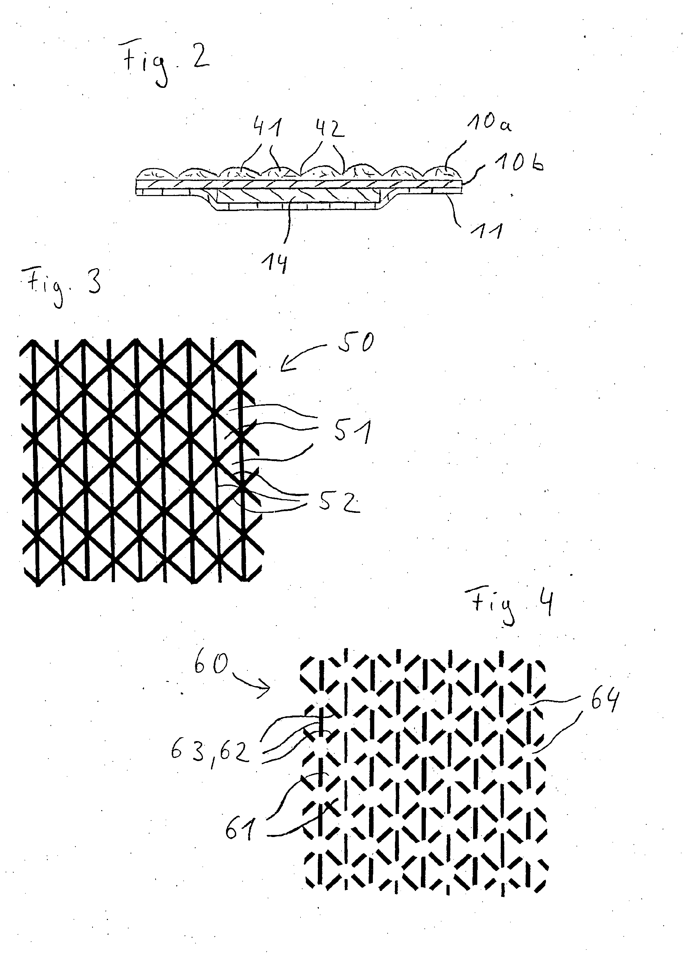

Die Außenseite des Backsheets 10 des Hauptteils 4 ist durch eine Backsheetvliesstoffkomponente 10a gebildet. In der nicht maßstabsgerechten Figur 2 ist erkennbar, dass das Backsheet aus einem Vliesfolienlaminat gebildet ist, das heißt zwischen dem Saugkörper 14 und der Backsheetvliesstoffkomponente 10a ist eine Backsheetfolienkomponente 10b angeordnet. Schematisch sind in Figur 2 außerdem die Fügebereiche 42 und die Schlaufenbereiche 41 des Prägemusters 40 dargestellt. Auch die Backsheetvliesstoffkomponente 10a ist im dargestellten Falle aus einem PP-Spinnvlies, allerdings von 20 g/m² gebildet. Die Faserstärke beträgt 2 dtex. Die Außenseite dieses Spinnvliesstoffes weist ein - in Figur 1 nur bereichsweise schematisch angedeutetes - Prägemuster 40 auf. Tatsächlich erstreckt sich das Prägemuster 40 über die gesamte Außenseite des Hauptteils 4 vom Vorder- 6 über den Schritt- 12 bis zum Rückbereich 8 der Inkontinenzwindel 2. Die durch Heißkalanderprägung erzeugten Fügebereiche sind durch eine Vielzahl von Linien gebildet, die sich zur Ausbildung eines regelmäßigen Rautenmusters schneiden, so dass inselartig angeordnete unverbundene, rautenförmige Schlaufenbereiche 41 von linienartigen Fügebereichen 42 umgeben sind. Im dargestellten Fall ist das Prägemuster 40 der Backsheetvliesstoffkomponente 10a dem des Prägemusters 20 der Vliesstoffkomponente der vorderen Seitenteile 16 identisch.

Die Über-Bauch-Haltekräfte zwischen den Verschlussmitteln 32 und der Außenseite der Seitenteile 16 betragen vorzugsweise mindestens 58 N/25mm und sind höher und als die Über-Bauch-Haltekräfte zwischen den Verschlussmitteln 32 und der Außenseite der Backsheetvliesstoffkomponente, die vorzugsweise gleichwohl mindestens 20 N/25mm betragen. Im Wesentlichen wird dies durch das höhere Flächengewicht der Vliesstoffkomponente der Seitenteile sichergestellt.

Figur 3 zeigt schematisch ein alternatives durch Heißkalanderung erzeugtes Prägemuster 50 für die Vliesstoffkomponenten der vorderen Seitenteile 16 und/oder des Backsheets 10. Das Prägemuster 50 weist Fügebereiche auf, die durch eine Vielzahl von Linien gebildet sind und die sich zur Ausbildung eines regelmäßigen Musters schneiden, so dass inselartig angeordnete unverbundene, dreieckförmige Schlaufenbereiche 51 von linienartigen Fügebereichen 52 umgeben sind. Die Prägefläche, also die Summe der Fläche aller Fügebereiche 52 bezogen auf die Gesamtfläche des durch das Prägemuster 50 erfassten Bereiches des Vliesstoffes beträgt in diesem Fall 23 %.

Figur 4 zeigt schematisch und nicht maßstabsgerecht eine Abwandlung des in Figur 3 dargestellten Prägemusters 50. In diesem Fall ist das Prägemuster ein offenes Prägemusters 60. Die Fügebereiche 62 umgeben die dreieckförmigen Schlaufen bereiche 61 nicht kontinuierlich also nicht lückenlos, sondern die Fügebereiche 62 sind aus einer Vielzahl von insbesondere kleineren Abschnitten linienförmiger Fügestellen 63 gebildet, die keine Schnittpunkte miteinander aufweisen; die Fügebereiche 62 sind also mit Durchbrechungen 64 versehen. Dies führt zu insgesamt besseren taktilen Eigenschaften, insbesondere zu höherer Weichheit einer mit diesem Prägemuster 60 versehenen Vliesstoffkomponente. Die linienförmigen Fügestellen 63 bilden jeweils die sich nicht treffenden Schenkel eines Dreiecks. In einer bevorzugten Ausführungsform des offenen Prägemuster 60 weisen die linienförmigen Fügestellen 63 eine Länge von 3-7 mm, insbesondere 4-5 mm auf. Vorzugsweise weisen die Fügestellen 63 eine einheitliche Länge auf, so dass sie eine Vielzahl von gleichseitigen Dreiecken bilden. Die Breite der Fügestellen 63 beträgt vorzugsweise 0,2-0,8 mm, insbesondere 0,4-0,6 mm. Die Prägetiefe beträgt vorzugsweise 0,2-1,0 mm, insbesondere 0,4-0,8 mm. Die Prägefläche, also die Summe der Fläche aller Fügebereiche 62 bezogen auf die Gesamtfläche des durch das Prägemuster 60 erfassten Bereiches beträgt vorzugsweise 15-50%, insbesondere 17-40 %, weiter insbesondere 19-25%. Im in Figur 4 dargestellten Fall beträgt die Prägefläche etwa 21 %.

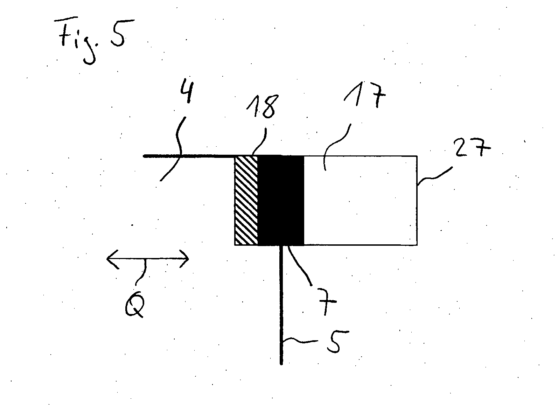

Figur 5 zeigt ausschnittsweise den Rückbereich einer erfindungsgemäßen Ausbildung der Inkontinenzwindel 2, wobei das schematisch dargestellte hintere Seitenteil 17 ein Verstärkungsmittel 7 aufweist, welches in Querrichtung Q schmäler ist als das Seitenteil 17. Das Verstärkungsmittel 7 erstreckt sich jedoch in Querrichtung Q über den Seitenrand 5 des Hauptteils 4 hinaus. Das Verstärkungsmittel 7 überfängt dabei den Überlappungsbereich 18 teilweise, wie schematisch in Figur 5 dargestellt ist. Es erstreckt sich also sowohl über den seitlichen Seitenrand 5 in Richtung freies Ende 27 des Seitenteils 17 als auch in Richtung Überlappungsbereich 18, also in Richtung einer Längsmittellinie L des Hauptteils 4.

Das Verstärkungsmittel 7 kann auf verschiedene Weise ausgebildet sein, solange es einen Einreißschutz des Seitenabschnitts 17 bewirkt, insbesondere bei Einleitung einer zur Querrichtung Q schräg gerichteten Zugkraft auf das Seitenteil 17 bzw. den Überlappungsbereich 18 beim Schließen der Windel mittels der in Figur 5 nicht dargestellten Verschlussstreifen 30. Das Verstärkungsmittel 7 kann beispielsweise von einem zusätzlichen Verstärkungsabschnitt, beispielsweise aus Vlies oder Folie oder aus einem an sich beliebigen verstärkenden Material gebildet sein. Dieses kann durch an sich beliebige Fügeverfahren, insbesondere unter Verwendung eines Klebers, auf das Material des Seitenabschnitts 17 aufgebracht sein.

In einer weiteren nicht dargestellten Ausführungsform ist das Verstärkungsmittel 7 von dem Material des Seitenteils 17 selbst gebildet ist, indem das Seitenteil 17 auf sich selbst, insbesondere z-förmig gefaltet ist, wobei sich die gefaltete Konfiguration in Querrichtung Q über den Seitenrand 5 des Hauptteils 4 in Richtung freies Ende des Seitenteils 17 hinaus erstreckt.

Eine besonders bevorzugte Inkontinenzwindel 2 weist folgende, bezüglich der Über-Bauch-Haltekräfte relevante Komponenten auf:

als vordere Seitenteile 16:

Material X: bestehend aus 30 g/m² Polypropylenspinnvlies, Faserstärke 2 dtex Heißkalanderprägemuster auf der Außenseite des Seitenteilmaterials nach Figur 4, wobei die Fügestellen 63 eine gleichförmige Länge von 4,5 mm aufweisen bei einer Breite der Fügestellen von 0,4 mm und bei einer Prägetiefe von 0,65 mm und bei einer Prägefläche von 21,13%. Das Material ist erhältlich bei Fa. Corovin GmbH, Wohltorfer Str. 124, D-31201 Peine.

als Backsheet 10:

Material Y: Eingesetzt wurde ein Vliesfolienlaminat, welches vorzugsweise die komplette Außenseite des Hauptteils 4 der Inkontinenzwindel 2 bildet. Die Vliesstoffkomponente besteht aus einem 20 g/m² Polypropylenspinnvlies, Faserstärke 2 dtex mit einem Heißkalanderprägemuster wie das der vorderen Seitenteile 16, also einem Heißkalanderprägemuster nach Figur 4, wobei die Fügestellen 63 eine gleichförmige Länge von 4,5 mm aufweisen bei einer Breite der Fügestellen von 0,4 mm und bei einer Prägetiefe von 0,65 mm und bei einer Prägefläche von 21,13%, zu beziehen bei der Fa. Corovin GmbH, Wohltorfer Str. 124, D-31201 Peine. Die Folienkomponente ist eine blasextrudierte Folie erhältlich bei der Fa. Rheinische Kunststoffwerke GmbH, Alkorstrasse 6, D-83512 Wasserburg. Die Rezeptur der Folie besteht aus 70 % niedrigschmelzendem Polypropylen-Compound (Schmelzpunkt ca. 137 - 143 °C) und 30 % hochschmelzendem Polypropylen-Compound (Schmelzpunkt 158 - 164 °C). Die Compounds bestehen jeweils aus einer Abmischung Polymer-Rohstoff plus 60 % CaCO₃ (Kreide). Nach der Blasextrusion der sogenannten Precursorfolie mit einem Flächengewicht von 40 g/m² wurde die Folie über eine monoaxiale MDO-Reckanlage in Maschinenrichtung gereckt. Dabei wurde die Folienbahn mit einem Reckgrad von 1:2 in Maschinenrichtung, also auf ein Flächengewicht von 20 g/m², gereckt und damit die Atmungsaktivität erzeugt. Die atmungsaktive Folie wurde anschließend gemeinsam mit dem Polypropylenspinnvlies (20 g/m²) in einer Vorrichtung, wie sie in Figur 1 der DE102004042405A1 offenbart und beschrieben ist bei 130 bis 140 °C thermolaminiert. Nachfolgend wurde das erzeugte Vliesfolienlaminat einem Ringrolling in CD (quer zur Maschinenrichtung) unterzogen. Dieses Laminat wurde anschließend der Heißtemperung zugeführt. Das Laminat lief hierbei über zwei hintereinander angeordnete Heizwalzen, wobei es auf eine Temperatur oberhalb der Schmelztemperatur der Folie, aber unterhalb der Schmelztemperatur des Polypropylenspinnvlieses erwärmt wurde, nämlich auf 130-140 °C. Nach der zweiten Heißwalze wurde das Laminat unmittelbar über zwei nacheinander angeordnete Kühlwalzen geführt. Diese hatten eine um so viel geringere Umlaufgeschwindigkeit als die Heizwalzen, wie der gewünschte Schrumpf in Maschinenrichtung beträgt, vorliegend ca. um 5%.

als mechanische Verschlusshilfen 31 aufweisende Verschlussmittel 32

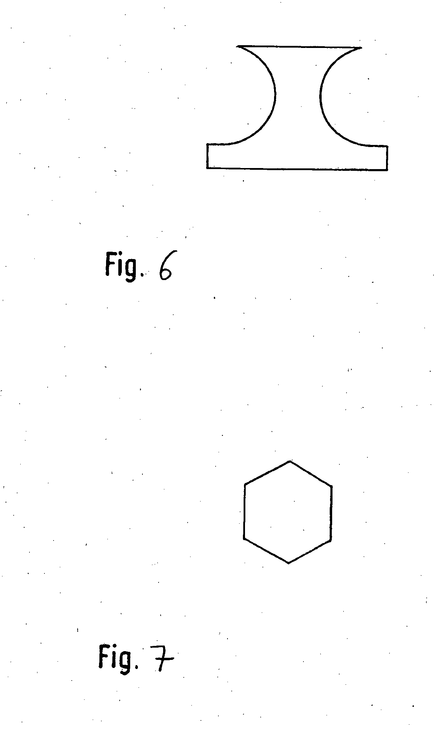

Material Z: Kletthakenabschnitt mit den Abmessungen 20 x 25 mm. Typ Microplast® 42-288-HX200-PP3, Artikel-Nummer 25445, erhältlich bei der Fa. G. Binder GmbH & Co KG Textil- und Kunststofftechnik, Holzgerlingen, Bundesrepublik Deutschland. Die Darstellung der Form dieser Kletthaken ist in Figuren 6 und 7 einzusehen. Die Haken haben eine pilzförmige Gestalt (Figur 6) mit hexagonaler Fläche des Kopfes 8 (Figur 7). Die Hakendichte (Anzahl Haken pro Fläche) beträgt 288 Haken pro cm². Das Material besteht aus Polypropylen und weist eine Dicke von 0,42 mm auf. Es ist durch Extrusion hergestellt. Die Höhe der pilzförmigen Erhebungen gegenüber dem Grund des Materials beträgt 0,26 mm. Der Abstand der Kanten der Hakenköpfe beträgt 200 μm.

Die Über-Bauch-Haftkräfte dieser Materialien ermittelt als Scherkräfte nach oben beschriebener Prüfmethode sind wie folgt:

Außenseite (Prägemusterseite) des Materials X mit Material Z: 65,3 N/25mm
Außenseite (Vliesstoffseite) des Materials Y mit Material Z: 48,6 N/25mm

Die Über-Bauch-Haltekräfte der Verschlussmittel mit der Außenseite des Seitenteilmaterials sind also höher als die Über-Bauch-Haltekräfte der Verschlussmittel mit der Außenseite des Backsheet bildenden Vliesfolienlaminates.

**Patentansprüche**

1.  Absorbierende Inkontinenzwindel (2) mit einem Rückbereich (8), einem Vorderbereich (6) und einem dazwischen liegenden Schrittbereich (12) und mit erstem und zweitem Seitenrand (5) und mit einem Hauptteil (4) mit einer in Gebrauch der Windel dem Körper zugewandten Innenseite und einer dem Körper abgewandten Außenseite, wobei der Hauptteil (4) einen Saugkörper (14) und ein Backsheet (10) auf der dem Körper abgewandten Seite des Saugkörpers (14) umfasst, und wobei der Saugkörper (14) eine geringere Breite aufweist als das Backsheet (10), und mit an den ersten und zweiten Seitenrand (5) angefügten Seitenteilen (16, 17), wobei die Inkontinenzwindel (2) vier diskrete nicht direkt miteinander verbundene Seitenteile (16, 17) aufweist, derart dass je ein Seitenteil an beiden Seitenrändern (5) des Vorderbereichs (6) und je ein weiteres Seitenteil an beiden Seitenrändern (5) des Rückbereichs (8) angefügt ist und wobei die Seitenteile (16, 17) eine Innenseite und eine Außenseite aufweisen und wobei die Seitenteile (17) im Rückbereich Verschlussmittel (32) mit mechanischen Verschlusshilfen (31) aufweisen und wobei die mechanischen Verschlusshilfen (31) Kletthaken umfassen und wobei die Verschlussmittel (32) zum bestimmungsgemäßen Festlegen der Windel an den Körper eines Menschen zumindest bereichsweise sowohl an der Außenseite des Hauptteils (4) als auch an der Außenseite der Seitenteile (16) im Vorderbereich (6) lösbar festlegbar sind und wobei die Haltekräfte zwischen den Verschlussmitteln (32) und der Außenseite des Hauptteils geringer sind als die Haltekräfte zwischen den Verschlussmitteln (32) und der Außenseite der Seitenteile (16) im Vorderbereich (6).

2.  Absorbierende Inkontinenzwindel (2) nach Anspruch 1, **dadurch gekennzeichnet, dass** die Haltekräfte als Über-Bauch-Haltekräfte ermittelt zwischen den mechanische Verschlusshilfen (31) aufweisenden Verschlussmitteln (32) und der Außenseite des Hauptteils 57-20 N/25mm, insbesondere 50-25 N/25mm betragen.

3.  Absorbierende Inkontinenzwindel (2) nach einem der vorstehenden Ansprüche, **dadurch gekennzeichnet, dass** die Haltekräfte als Über-Bauch-Haltekräfte ermittelt zwischen den mechanische Verschlusshilfen (31) aufweisenden Verschlussmitteln (32) und der Außenseite der Seitenteile im Vorderbereich 90-58 N/25mm, insbesondere 80-60 N/25mm betragen.

4.  Absorbierende Inkontinenzwindel (2) nach einem der vorstehenden Ansprüche, **dadurch gekennzeichnet, dass** zumindest ein Verschlussmittel (32) außerdem eine klebende Verschlusshilfe, insbesondere eine Haftklebezone umfasst.

5.  Absorbierende Inkontinenzwindel (2) nach einem der vorstehenden Ansprüche, **dadurch gekennzeichnet, dass** die Außenseite des Hauptteils zumindest bereichsweise, insbesondere aber vollflächig durch einen Vliesstoff gebildet ist.

6.  Absorbierende Inkontinenzwindel (2) nach einem der vorstehenden Ansprüche, **dadurch gekennzeichnet, dass** das Backsheet (10) aus einem Vliesfolienlaminat gebildet ist, wobei der Vliesstoff (10a) außen und die Folien (10b) innen zum Saugkörper (14) gerichtet zu liegen kommt, so dass der Vliesstoff die Außenseite des Hauptteils (4) bildet.

7.  Absorbierende Inkontinenzwindel (2) nach einem der Ansprüche 5-6, **dadurch gekennzeichnet, dass** das Flächengewicht des Vliesstoffes 10-30 g/m$^2$, insbesondere 14-25 g/m$^2$ und weiter insbesondere 18-22 g/m$^2$ beträgt.

8.  Absorbierende Inkontinenzwindel (2) nach Anspruch 6, **dadurch gekennzeichnet, dass** die Folie aus einer ein- oder mehrschichtigen flüssigkeitsundurchlässigen aber gleichwohl atmungsaktiven Folie gebildet ist.

9.  Absorbierende Inkontinenzwindel (2) nach einem der Ansprüche 5-8, **dadurch gekennzeichnet, dass** der Vliesstoff oder das Vliesfolienlaminat zur Verbesserung der Über-Bauch-Haltekräfte einer Temperaturbehandlung oberhalb von 60 °C unterzogen wurde.

10. Absorbierende Inkontinenzwindel (2) nach einem der Ansprüche 5-9, **dadurch gekennzeichnet, dass** der Vliesstoff oder das Vliesfolienlaminat zur Verbesserung der Über-Bauch-Haltekräfte einem Ring-Rolling ausgesetzt wurde.

**11.** Absorbierende Inkontinenzwindel (2) nach einem der vorstehenden Ansprüche, **dadurch gekennzeichnet, dass** die Außenseiten der Seitenteile (16, 17) im Vorder- und/oder im Rückbereich durch einen Vliesstoff gebildet sind.

**12.** Absorbierende Inkontinenzwindel (2) nach einem der vorstehenden Ansprüche, **dadurch gekennzeichnet, dass** die Seitenteile (16, 17) im Vorder- und/oder im Rückbereich durch einen luft- und wasserdampfdurchlässigen Vliesstoff gebildet sind.

**13.** Absorbierende Inkontinenzwindel (2) nach einem der vorstehenden Ansprüche, **dadurch gekennzeichnet, dass** das Flächengewicht der Seitenteile (16, 17) im Vorder- und/oder im Rückbereich 18-60 g/m$^2$; insbesondere 25-45 g/m$^2$, weiter insbesondere 27-40 g/m$^2$ und weiter insbesondere 28-35 g/m$^2$ beträgt.

**14.** Absorbierende Inkontinenzwindel (2) nach einem der vorstehenden Ansprüche, **dadurch gekennzeichnet, dass** die Atmungsaktivität der Seitenteile (16, 17) im Vorder- und/oder im Rückbereich größer ist als die Atmungsaktivität des Backsheets (10).

**15.** Absorbierende Inkontinenzwindel (2) nach einem der vorstehenden Ansprüche, **dadurch gekennzeichnet, dass** die Seitenteile (16, 17) im Vorder- und/oder im Rückbereich wenigstens um eine in Längsrichtung verlaufende Falzlinie auf sich selbst gefaltet sind.

**16.** Absorbierende Inkontinenzwindel (2) nach einem der vorstehenden Ansprüche, **dadurch gekennzeichnet, dass** aufeinander gefaltete und flächenhaft aneinander liegende Teilabschnitte der Seitenteile (16, 17) in dieser gefalteten Konfiguration an Fügestellen oder Fügebereichen lösbar aneinander fixiert sind.

**17.** Absorbierende Inkontinenzwindel (2) nach einem der vorstehenden Ansprüche, **dadurch gekennzeichnet, dass** die Seitenteile (16, 17) im Vorder- und/oder im Rückbereich ein Verstärkungsmittel (7) aufweisen, welches in Querrichtung (Q) betrachtet schmäler ausgebildet ist als ein jeweiliges Seitenteil (16, 17) und welches wenigstens in einem den Seitenrand (5) des Hauptteils (4) überbrückenden Bereich vorgesehen ist, also sowohl einen seitlichen Seitenrandbereich des Hauptteils (4) als auch einen Teil des Seitenteils (16, 17) in Querrichtung (7) überfängt.

**Claims**

**1.** Absorbent incontinence diaper (2) having a rear region (8), a front region (6) and an intermediate crotch region (12), and having a first and second side periphery (5) and having a main part (4) having an inner side that faces the body when the diaper is in use, and an outer side that faces away from the body, wherein the main part (4) comprises an absorbent body (14) and backsheet (10) on that side of the absorbent body (14) that faces away from the body, and wherein the absorbent body (14) has a smaller width than the backsheet (10), and having side parts (16, 17) attached to the first and second side peripheries (5), wherein the incontinence diaper (2) has four discrete side parts (16, 17) that are not connected directly together, such that a respective side part is attached to each of the two side peripheries (5) of the front region (6) and a further respective side part is attached to each of the two side peripheries (5) of the rear region (8), and wherein the side parts (16, 17) have an inner side and an outer side, and wherein the side parts (17) in the rear region have closure means (32) with mechanical closure aids (31), and wherein the mechanical closure aids (31) comprise hooks, and wherein, in order to secure the diaper to the body of a person properly, the closure means (32) are releasably securable at least regionally both on the outer side of the main part (4) and on the outer side of the side parts (16) in the front region (6), and wherein the retaining forces between the closure means (32) and the outer side of the main part are less than the retaining forces between the closure means (32) and the outer side of the side parts (16) in the front region (6).

**2.** Absorbent incontinence diaper (2) according to Claim 1, **characterized in that** the retaining forces, determined as over-the-stomach retaining forces, are 57-20 N/25 mm, in particular 50-25 N/25 mm, between the closure means (32) having mechanical closure aids (31) and the outer side of the main part.

**3.** Absorbent incontinence diaper (2) according to either of the preceding claims, **characterized in that** the retaining forces, determined as over-the-stomach retaining forces, are 90-58 N/25 mm, in particular 80-60 N/25 mm, between the closure means (32) having mechanical closure aids (31) and the outer side of the side parts in the front region.

**4.** Absorbent incontinence diaper (2) according to one of the preceding claims, **characterized in that** at least one closure means (32) also comprises an adhesive closure aid, in particular a pressure-sensitive adhesive zone.

**5.** Absorbent incontinence diaper (2) according to one of the preceding claims, **characterized in that** the outer side of the main part is formed at least regionally, but in particular over its entire area, by a nonwoven material.

6. Absorbent incontinence diaper (2) according to one of the preceding claims, **characterized in that** the backsheet (10) is formed from a nonwoven-film laminate, wherein the nonwoven material (10a) comes to lie externally and the film (10b) internally, directed towards the absorbent body (14), such that the nonwoven material forms the outer side of the main part (4).

7. Absorbent incontinence diaper (2) according to either of Claims 5 and 6, **characterized in that** the weight per unit area of the nonwoven material is 10-30 g/m$^2$, in particular 14-25 g/m$^2$ and more particularly 18-22 g/m$^2$.

8. Absorbent incontinence diaper (2) according to Claim 6, **characterized in that** the film is formed from a single-layer or multilayer liquid-impermeable but nevertheless breathable film.

9. Absorbent incontinence diaper (2) according to one of Claims 5-8, **characterized in that** the nonwoven material or the nonwoven-film laminate has been subjected to a heat treatment at above 60°C in order to improve the over-the-stomach retaining forces.

10. Absorbent incontinence diaper (2) according to one of Claims 5-9, **characterized in that** the nonwoven material or the nonwoven-film laminate has been subjected to ring-rolling in order to improve the over-the-stomach retaining forces.

11. Absorbent incontinence diaper (2) according to one of the preceding claims, **characterized in that** the outer sides of the side parts (16, 17) in the front region and/or in the rear region are formed by a nonwoven material.

12. Absorbent incontinence diaper (2) according to one of the preceding claims, **characterized in that** the side parts (16, 17) in the front region and/or in the rear region are formed by an air- and water-vapour-permeable nonwoven material.

13. Absorbent incontinence diaper (2) according to one of the preceding claims, **characterized in that** the weight per unit area of the side parts (16, 17) in the front region and/or in the rear region is 18-60 g/m$^2$, in particular 25-45 g/m$^2$, more particularly 27-40 g/m$^2$ and more particularly 28-35 g/m$^2$.

14. Absorbent incontinence diaper (2) according to one of the preceding claims, **characterized in that** the breathability of the side parts (16, 17) in the front region and/or in the rear region is greater than the breathability of the backsheet (10).

15. Absorbent incontinence diaper (2) according to one of the preceding claims, **characterized in that** the side parts (16, 17) in the front region and/or in the rear region are folded over themselves at least about a fold line extending in the longitudinal direction.

16. Absorbent incontinence diaper (2) according to one of the preceding claims, **characterized in that** subportions, folded one over another and being in surface contact with one another, of the side parts (16, 17) are releasably fixed together in this folded configuration at joining points or in joining regions.

17. Absorbent incontinence diaper (2) according to one of the preceding claims, **characterized in that** the side parts (16, 17) in the front region and/or in the rear region have a reinforcing means (7) which is configured in a narrower manner, as seen in the transverse direction (Q), than a respective side part (16, 17) and which is provided at least in a region bridging the side periphery (5) of the main part (4), that is to say overlays both a lateral side-periphery region of the main part (4) and a part of the side part (16, 17) in the transverse direction (Q).

**Revendications**

1. Couche absorbante pour incontinence (2) comprenant une région arrière (8), une région avant (6) et une région de fourche (12) située entre elles et comprenant un premier et un deuxième bord latéral (5) et une partie principale (4) avec un côté intérieur tourné vers le corps pendant l'utilisation de la couche et un côté extérieur opposé au corps, la partie principale (4) comprenant un corps absorbant (14) et une feuille de support (10) du côté du corps absorbant (14) opposé au corps, et le corps absorbant (14) présentant une plus faible largeur que la feuille de support (10), et comprenant des parties latérales (16, 17) jointes au premier et au deuxième bord latéral (5), la couche pour incontinence (2) présentant quatre parties latérales (16, 17) distinctes non connectées directement les unes aux autres de telle sorte qu'une partie latérale soit à chaque fois jointe aux deux bords latéraux (5) de la région avant (6) et qu'une partie latérale supplémentaire soit à chaque fois jointe aux deux bords latéraux (5) de la région arrière (8) et les parties latérales (16, 17) présentant un côté intérieur et un côté extérieur et les parties latérales (17) présentant, dans la région arrière, des moyens de fermeture (32) avec des auxiliaires de fermeture mécaniques (31) et les auxiliaires de fermeture mécaniques (31) comprenant des crochets pour fermeture agrippante et les moyens de fermeture (32) pouvant être fixés de manière amovible pour la fixation correcte de la couche au corps d'une personne au moins en partie à la fois au niveau du côté extérieur de la partie principale (4) et au niveau du côté extérieur des parties latérales (16) dans la région avant (6) et les forces de retenue entre les moyens de fermeture (32) et le côté extérieur de la partie principale étant inférieures aux forces de retenue entre les moyens de fermeture (32) et le côté extérieur des parties latérales (16) dans la région avant (6).

**2.** Couche absorbante pour incontinence (2) selon la revendication 1, **caractérisée en ce que** les forces de retenue, déterminées sous la forme de forces de retenue au-dessus du ventre entre les moyens de fermeture (32) présentant des auxiliaires de fermeture mécaniques (31) et le côté extérieur de la partie principale, valent 57-20 N/25 mm, en particulier 50-25 N/25 mm.

**3.** Couche absorbante pour incontinence (2) selon l'une quelconque des revendications précédentes, **caractérisée en ce que** les forces de retenue, déterminées sous la forme de forces de retenue au-dessus du ventre entre les moyens de fermeture (32) présentant des auxiliaires de fermeture mécaniques (31) et le côté extérieur des parties latérales dans la région avant, valent 90-58 N/25 mm, en particulier 80-60 N/25 mm.

**4.** Couche absorbante pour incontinence (2) selon l'une quelconque des revendications précédentes, **caractérisée en ce qu'**au moins un moyen de fermeture (32) comprend en outre un auxiliaire de fermeture adhésif, en particulier une zone autocollante.

**5.** Couche absorbante pour incontinence (2) selon l'une quelconque des revendications précédentes, **caractérisée en ce que** le côté extérieur de la partie principale est formé au moins en partie, mais notamment sur toute la surface, par un matériau non-tissé.

**6.** Couche absorbante pour incontinence (2) selon l'une quelconque des revendications précédentes, **caractérisée en ce que** la feuille de support (10) est formée par un stratifié en feuille et matériau non-tissé, le matériau non-tissé (10a) venant s'appliquer à l'extérieur et les feuilles (10b) venant s'appliquer à l'intérieur dans la direction du corps absorbant (14) de sorte que le matériau non-tissé forme le côté extérieur de la partie principale (4).

**7.** Couche absorbante pour incontinence (2) selon l'une quelconque des revendications 5 à 6, **caractérisée en ce que** le grammage du matériau non-tissé vaut 10-30 g/m², en particulier 14-25 g/m² et plus particulièrement 18-22 g/m².

**8.** Couche absorbante pour incontinence (2) selon la revendication 6, **caractérisée en ce que** la feuille est formée d'une feuille monocouche ou multicouche imperméable aux liquides mais tout en étant respirante.

**9.** Couche absorbante pour incontinence (2) selon l'une quelconque des revendications 5 à 8, **caractérisée en ce que** le matériau non-tissé ou le stratifié en feuille et matériau non-tissé a été soumis à un traitement thermique au-dessus de 60°C pour améliorer les forces de retenue au-dessus du ventre.

**10.** Couche absorbante pour incontinence (2) selon l'une quelconque des revendications 5 à 9, **caractérisée en ce que** le matériau non-tissé ou le stratifié en feuille et matériau non-tissé a été soumis à un laminage au rouleau pour améliorer les forces de retenue au-dessus du ventre.

**11.** Couche absorbante pour incontinence (2) selon l'une quelconque des revendications précédentes, **caractérisée en ce que** les côtés extérieurs des parties latérales (16, 17) sont formés dans la région avant et/ou dans la région arrière par un matériau non-tissé.

**12.** Couche absorbante pour incontinence (2) selon l'une quelconque des revendications précédentes, **caractérisée en ce que** les parties latérales (16, 17) dans la région avant et/ou dans la région arrière sont formées par un matériau non-tissé perméable à l'air et à la vapeur d'eau.

**13.** Couche absorbante pour incontinence (2) selon l'une quelconque des revendications précédentes, **caractérisée en ce que** le grammage des parties latérales (16, 17) dans la région avant et/ou dans la région arrière vaut 18-60 g/m², en particulier 25-45 g/m², plus particulièrement 27-40 g/m² et plus particulièrement 28-35 g/m².

**14.** Couche absorbante pour incontinence (2) selon l'une quelconque des revendications précédentes, **caractérisée en ce que** l'activité respirante des parties latérales (16, 17) dans la région avant et/ou dans la région arrière est plus importante que l'activité respirante de la feuille de support (10).

**15.** Couche absorbante pour incontinence (2) selon l'une quelconque des revendications précédentes, **caractérisée en ce que** les parties latérales (16, 17) dans la région avant et/ou dans la région arrière sont repliées sur elles-mêmes au moins autour d'une ligne de pliage s'étendant dans la direction longitudinale.

**16.** Couche absorbante pour incontinence (2) selon l'une quelconque des revendications précédentes, **caractérisée en ce que** des sections partielles des parties latérales (16, 17) pliées les unes sur les autres et situées avec leurs surfaces à plat les unes contre les autres sont fixées les unes aux autres de manière amovible dans cette configuration pliée au niveau de points de jonction ou de régions de jonction.

17. Couche absorbante pour incontinence (2) selon l'une quelconque des revendications précédentes, **caractérisée en ce que** les parties latérales (16, 17) dans la région avant et/ou dans la région arrière présentent un moyen de renforcement (7) qui, considéré dans la direction transversale (Q), est plus étroit qu'une partie latérale respective (16, 17) et qui est prévu au moins dans une région surmontant le bord latéral (5) de la partie principale (4), c'est-à-dire qui recouvre à la fois une région latérale de bord latéral de la partie principale (4) et une partie de la partie latérale (16, 17) dans la direction transversale (Q).

Fig. 1

Fig. 2

Fig. 3

Fig. 4

Fig. 5

Fig. 6

Fig. 7

Fig. 8

Fig. 9b

141

141

106

101

101

108

Fig. 9a

300mm

10mm

25mm

110

108

20mm

50mm

50mm

106

EP 1 915 977 B1

22

Fig. 9 c

Fig. 9 d

104

100

102

104

**Fig. 10**

Fig. 11a

A

30

31,32

B

Fig. 11b

A

30

31    33

32

B

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004105668 A1
- EP 0882828 A1
- DE 102004053469 A1
- DE 102004042405 A1
- DE 102004021353 A1